# EUROPEAN PATENT APPLICATION

(11) **EP 3 689 367 A1**
(43) Date of publication of application: **05.08.2020**
(21) Application number: 19154849.4
(22) Date of filing: 31.01.2019
(51) Int. Cl.: A61K 38/39, A61K 35/39, A61P 3/10

(54) **IMPROVED MEANS AND METHODS TO TREAT DIABETES**

(71) Applicant: Eberhard Karls Universität Tübingen Medizinische Fakultät, 72074 Tübingen (DE)
(72) Inventor: Layland, Shannon, 72070 Tübingen (DE); Schenke-Layland, Katja, 72070 Tübingen (DE); Duffy, Garry, Kildare (IE); Hinderer, Svenja, 72631 Aichtal (DE); Zbinden, Aline, 72076 Tübingen (DE)
(74) Representative: Witte, Weller & Partner Patentanwälte mbB

(57) **Abstract**

The present application relates to a pharmaceutical composition for use in a method of treating diabetes in a human or animal subject in need thereof, to transplantation devices comprising the pharmaceutical composition of the present invention and to methods of treating diabetes using the pharmaceutical composition and transplantation device of the present invention.

## Description

The present application relates to a pharmaceutical composition for use in a method of treating diabetes in a human or animal subject in need thereof, to transplantation devices comprising the pharmaceutical composition of the present invention and to methods of treating diabetes using the pharmaceutical composition and transplantation device of the present invention.

Diabetes mellitus is a chronic disease characterized by high blood glucose due to inadequate insulin production and/or insulin resistance. It affects around 400 million people worldwide and causes an estimated 5 million deaths per year. Type I diabetes (T1D) is a permanent disease characterized by the autoimmune destruction of the insulin-secreting β-cells within the pancreatic islets. Type II diabetes (T2D) is caused by the stress-induced loss of β-cells due to the constant demand to produce insulin caused by persistent hyperglycemia.

The prevention and treatment of diabetes regularly involves maintaining a healthy diet, maintaining a normal body weight, exercising, surveying various other body parameters and, of course treatment with insulin.

Currently, exogenous insulin administration still is the golden standard and provides effective management of T1D and insulin-dependent T2D, but requires the constant monitoring of blood glucose to prevent hypoglycemia. Alternatively, β-cell-containing islet transplantation, known as the Edmonton protocol, can re-establish the body's natural regulated insulin production upon glucose stimulation.

US 2008/0103606 A1 discloses an implantable device containing individual islet cells or small islet cell clusters for diabetes treatment, while US 6,365,385 B1 discloses methods of culturing and encapsulating pancreatic islet cells. US 2005/0180957 A1 discloses methods of using fibrin-bound angiogenic factors to stimulate vascularisation at transplant sites of encapsulated cells. US 5,672,361 discloses the use of laminin-5 for the growth of pancreatic islet cells.

US 5,510,263 A discloses methods to grow pancreatic islet-like cell clusters. US 6,815,203 B1 discloses methods of obtaining pancreatic islet cells from dedifferentiated pancreatic cells using inter alia nidogen-1 (NID1) as extracellular cell matrix (ECM) component.

US 2014/0113347 A1 discloses various biopolymer compositions for encapsulating cells for cell-transplantation containing alginate, at least one glycosaminoglycan compound, which is preferably chondroitin sulfate, and at least one component of the extracellular matrix. The component of the extracellular matrix is preferably laminin but may be one or more of elastin, entactin-1, fibrillin, fibronectin, fibrin, fibrinogen, perlecan, heparin, heparan sulfate, decorin, keratin, syndecan, agrin, integrin, nidogen and others to be used with alginate and the at least one glycosaminoglycan component in a biopolymer composition for encapsulating cells, for instance liver cells, pancreatic cells, muscle cells, or other types of cells.

Cell-transplantation-based methods to treat diabetes are therefore known but are, however, inter alia severely limited by the short supply of donor pancreases. Other hurdles involved with islet transplants include sufficient delivery, retention, maintaining survival, engraftment and functioning of newly implanted islets. Reasons for islet graft loss include (1) hypoxia due to inadequate early vascularization, (2) insufficient retention due to lack of a suitable support matrix, (3) inadequate extracellular matrix (ECM) cues at the site of transplantation, (4) inflammatory reaction at the delivery site and (5) allo- and auto-rejection.

Thus, the prior art discloses various compositions employing extracellular matrix components and/or cells including pancreatic islets to treat glucose metabolism related diseases such as diabetes. However, there still exists the need to provide improved cell-transplantation-based methods and means for treating diabetes type I or II in a human or animal subject in need thereof.

The technical problem underlying the claimed invention is, thus, to provide means and methods for a cell-transplantation-based treatment of diabetes, in particular diabetes I and II, which at least partially overcome the above-identified disadvantages of the prior art, in particular which are improved means and methods to treat diabetes, in particular type I and II, based on cell transplantation, whereby one or more of an increased angiogenesis and/or vascularisation, an improved retention, an improved survival and an improved reinnervation of the transplanted cells can be achieved.

The present invention solves its underlying technical problem by providing a pharmaceutical composition for use in a method of treating diabetes, in particular diabetes type I or II, in a human or animal subject in need thereof comprising nidogen, in particular nidogen-1, a liquid or semiliquid pharmaceutically acceptable carrier and one or more of the cell components pancreatic islets, pancreatic pseudo-islets, pancreatic cells, non-pancreatic insulin producing cells and pancreatic β-cells, wherein nidogen, in particular the nidogen-1 is present in a concentration of from 25 µg/ml to 35 µg/ml in the pharmaceutical composition. It was surprisingly found that this specific range of concentrations of nidogen, in particular nidogen-1, leads to an increased survival and improved insulin production of cultured pancreatic cells in a hypoxic post-transplant environment. As mentioned above, one obstacle in cell transplantation methods is the lack of sufficient oxygen and/or nutrient supply of the transplanted cells because of e.g. absent vasculature leading to lowered metabolic activity and cell death. Advantageously, nidogen, in particular nidogen-1, as used in the present invention is able to increase angiogenesis and vascularisation of the transplanted cells as well as provide adequate extracellular matrix cues for the transplanted cells leading to increased retention and hence to a longer life-span of the transplanted cells.

Thus, the present invention provides a pharmaceutical composition which comprises nidogen, in particular nidogen-1, a pharmaceutically acceptable carrier, which is in liquid or semiliquid form and at least one cell component selected from the group consisting of pancreatic islets, pancreatic pseudo-islets, pancreatic cells, non-pancreatic insulin producing cells, and pancreatic β-cells, in particular two or more of said cell components, and wherein the nidogen, in particular nidogen-1, is present in a concentration of from 25 µg/ml to 35 µg/ml in the pharmaceutical composition.

The present invention in a preferred embodiment uses 27 to 33 µg/ml, preferably 28 to 31 µg/ml, preferably 29 to 30 µg/ml, preferably 30 to 31 µg/ml nidogen, in particular nidogen-1, in particular 30 µg/ml nidogen, in particular nidogen-1, in the pharmaceutical composition.

The pharmaceutical composition of the present invention is in particular for use in a method of treating diabetes, preferably diabetes type I or II, preferably in a human or animal subject in need thereof. The present invention therefore provides for the use of the protein nidogen, in particular nidogen-1 (NID1), also known as entactin, as a natural extracellular matrix biomaterial to biofunctionalise a composition, in particular a liquid or semiliquid pharmaceutically acceptable carrier, in particular a gel, a hydrogel, a suspension or a solution, containing particular cell components, namely selected from the group consisting of pancreatic islets, pancreatic pseudo-islets, pancreatic cells, non-pancreatic insulin producing cells and pancreatic β-cells, which biofunctionalised pharmaceutically acceptable carrier surrounds and protects said cell components before, during and after transplantation of said cell components into the subject in need thereof. The pharmaceutical composition of the present invention advantageously increases angiogenesis and vascularisation of the transplanted cells or the transplanted device containing the cells. The present invention provides the teaching to use nidogen-1 as a suitable support matrix for the cell components to be transplanted improving the survival of the transplanted cell components. Advantageously, according to the present invention nidogen-1 also supports the reinnervation of damaged tissue and therefore supports reinnervation of transplanted cell components. Thus, the present invention provides means and methods to improve the therapy of diabetes I and II based on a cell transplantation approach.

In the context of the present invention the term "diabetes" means diabetes mellitus, preferably diabetes mellitus type I, diabetes mellitus type II, gestational diabetes, pre-diabetes or latent autoimmune diabetes of adults (LADA) and congenital diabetes.

In the context of the present invention the terms "cell component", "cells" or "group of cells" refers to one or more of pancreatic islets, pancreatic pseudo-islets, pancreatic cells, non-pancreatic insulin producing cells, and pancreatic β-cells, in particular preparations of single cells, cell clusters, aggregates or separate units of specialized cells like e.g. pancreatic islets of said group. Such cell components are preferably isolated from adult human or animal donor tissue.

In the context of the present invention the term "pancreatic islets", also known as islets of Langerhans, refers to the regions of the pancreas that contain the endocrine, i.e. the hormone-producing cells and further to compositions containing pancreatic islets, in particular in isolated form thus containing the hormone-, in particular insulin-producing β-cells of the pancreas. In particular, a pancreatic islet is constituted by a thin fibrous connective tissue capsule surrounding the hormone-producing cells. The term "pancreatic islet" as used herein also comprises superstructures of pancreatic islets, also called islet clusters.

In the context of the present invention, the term "pancreatic pseudo-islets" means aggregates formed from islet cells having been obtained from pancreatic islets, for instance during the preparation of islets, for instance as cell suspensions, and re-aggregating the cells therein.

In the context of the present invention, the term "pancreatic cells" means cells occurring in the pancreas, in particular hormone-producing alpha-, beta-, delta-, PP- and epsilon-cells, preferably insulin-producing beta-cells.

In a preferred embodiment of the present invention, the pancreatic cells are cells occurring naturally in the pancreas. Preferably, the term "pancreatic cell" includes multipotent pancreatic progenitor cells, induced pluripotent stem cells able to produce insulin and pancreatic cells derived from embryonic stem cells.

In the context of the present invention, the term "non-pancreatic insulin producing cells" means cells from a non-pancreatic origin or without pancreatic cell characteristics which produce insulin.

In the context of the present invention, the term "pancreatic β-cells" means those cells in the pancreas, in particular pancreatic islets and pancreatic pseudo-islets, which are producing insulin or amylin, or both.

In the context of the present invention a human or animal subject is a human or animal patient, in particular a human or animal recipient of a cell transplant. In a preferred embodiment the human subject is a human. In a furthermore preferred embodiment the animal subject is a mammal, in particular a dog, cat or horse.

In the context of the present invention the terms "treatment" and "treating" mean obtaining a desired pharmaceutical and/or physiological effect. Preferably, the effect is therapeutic in terms of partially or completely curing diabetes in a subject, which means an achievement of at least partial or preferably complete independence from exogenous insulin and improved or restored metabolic control.

As used herein "nidogen", formerly called entactin, is a sulfated multidomain glycoprotein component of basement membranes, alongside other components such as collagen type IV, proteoglycans, such as heparan sulfate and glycosaminoglycans, laminin and fibronectin. Nidogen binds calcium ions and this calcium-binding activity of nidogen may play a role in the matrix assembly process. Its major function appears to be the assembly of the basement membrane. In particular, in the context of the present invention the term "nidogen" refers to a family of sulphated monomeric glycoproteins located in the basal lamina, in particular nidogen-1 (NID1) and nidogen-2 (NID2). Preferably, the nidogen used in the present invention is NID1. In a preferred embodiment of the present invention the term "nidogen" refers to the natural wild-type protein. The term "nidogen" may also refer to derivatives of nidogen, in particular forms with an altered amino acid and/or glycosylation structure, as long as they have the same or a comparable biological activity than wild-type nidogen. Furthermore preferred, the nidogen is a mammalian nidogen, preferably human or humanized nidogen.

According to the present invention the pharmaceutical composition provided comprises at least one of the cell components selected from the group consisting of pancreatic islets, pancreatic pseudo-islets, pancreatic cells, non-pancreatic insulin producing cells, and pancreatic β-cells. Accordingly, the pharmaceutical composition comprises at least one cell component with the ability to produce insulin. Additionally, further cells may be contained in the pharmaceutical composition which do not have the ability to produce insulin but which have a beneficial effect onto the cell components to be transplanted, for instance supporting their delivery, retention, survival or engraftment in the recipient. In a preferred embodiment of the present invention, the pharmaceutical composition comprises the above-identified cell components as the only cell components meaning that no other cell components, in particular no other cells are present in the pharmaceutical composition.

In a preferred embodiment of the present invention, the only cell component provided in the pharmaceutical composition are pancreatic islets. In a furthermore preferred embodiment of the present invention the only cell component provided in the pharmaceutical composition are pancreatic pseudo-islets. In a furthermore preferred embodiment of the present invention the only cell components contained in the pharmaceutical composition of the present invention are pancreatic cells. In a furthermore preferred embodiment of the present invention the only cell components contained in the pharmaceutical composition of the present invention are non-pancreatic insulin producing cells. In a furthermore preferred embodiment of the present invention the only cell components contained in the pharmaceutical composition of the present invention are pancreatic β-cells.

In a preferred embodiment of the present invention, the pharmaceutical composition contains a therapeutically effective amount of the one or more cell components for the treatment of diabetes. Preferably, this amount of the one or more cell component leads to an achievement of independence from exogenous insulin in the human or animal patient.

In a preferred embodiment of the present invention, the cell component used in the pharmaceutical composition of the present invention and in the present method of treating diabetes in a human or animal subject in need thereof are pancreatic islets.

In a preferred embodiment of the present invention, the cell component used in the pharmaceutical composition of the present invention and in the present method of treating diabetes in a human or animal subject in need thereof are pancreatic pseudo-islets.

In a preferred embodiment of the present invention, the cell component used in the pharmaceutical composition of the present invention and in the present method of treating diabetes in a human or animal subject in need thereof are pancreatic islets and pancreatic pseudo-islets.

In a preferred embodiment of the present invention, the therapeutically effective amount of the pancreatic islets, or pancreatic pseudo-islets or both is 1,000 (one thousand) to 70,000 (seventy thousand), in particular 4,000 to 60,000, preferably 5,000 to 40,000, preferably 6,000 to 35,000, preferably 7,000 to 30,000, preferably 8,000 to 25,000, preferably 9,000 to 13,000, in particular 11,000 islet equivalents (IE) per kg/bodyweight of the human or animal patient.

The number of islet equivalents can be determined according to the method as described in Assessment of Isolated Islet Equivalents M.P.M. van der Burg, et al. Transplantation Proceedings, 29, 1971-1973 (1997).

In a preferred embodiment of the present invention, the cell components used in the pharmaceutical composition of the present invention and the present method of treating diabetes in a human or animal subject in need thereof are pancreatic cells or non-pancreatic insulin-producing cells or pancreatic β-cells, or a combination thereof, in particular are pancreatic β-cells.

In preferred embodiments of the present invention, the pharmaceutical composition contains a therapeutically effective amount of the one or more cell component pancreatic cells or non-pancreatic insulin-producing cells or pancreatic β-cells or a combination thereof, in particular pancreatic β-cells, which is 1,000,000 (one million) to 90,000,000 (90 million), in particular 2,000,000 to 85,000,000, in particular 3,000,000 to 80,000,000, in particular 3,000,000 to 75,000,000, preferably 4,000,000 to 70,000,000, in particular 5.000.000 to 60,000,000, in particular 6,000,000 to 50,000,000, in particular 8,000,000 to 15,000,000, in particular 10,000,000 to 14,000,000, in particular at least 12,000,000, in particular 12,000,000, cells per kg/body weight of the human or animal patient.

In a furthermore preferred embodiment of the present invention, the pharmaceutical composition comprises nidogen, in particular nidogen-1. In a preferred embodiment further proteins may be contained in the pharmaceutical composition. However, in a furthermore preferred embodiment of the present invention nidogen is the only protein contained in the pharmaceutical composition, in particular in an isolated form, except those proteins which are present in the at least one cell component contained in the pharmaceutical composition as well.

In a furthermore preferred embodiment of the present invention nidogen, in particular nidogen-1, is present in the pharmaceutical composition in isolated, in particular pure and preferably non-denatured form.

In a preferred embodiment of the present invention the pharmaceutical composition comprises a semi-liquid or liquid pharmaceutical acceptable carrier which is preferably a solution, a suspension, a gel or hydrogel, in particular which is preferably present in the form of a solution, a suspension, a gel or a hydrogel.

In a preferred embodiment of the present invention the pharmaceutical composition comprises a liquid pharmaceutically acceptable carrier, in particular in the form of a solution or suspension. Pharmaceutically acceptable solutions or suspensions include saline, aqueous buffer solutions or solvents.

In a furthermore preferred embodiment of the present invention the pharmaceutical composition of the present invention comprises a semi-liquid pharmaceutically acceptable carrier, preferably in the form of a hydrogel or a gel.

In a preferred embodiment of the present invention, the nidogen and the one or more cell components are embedded in the pharmaceutical acceptable carrier which is preferably a hydrogel or gel.

In general, as used herein, the term "pharmaceutically acceptable carrier" refers to a diluent, adjuvant, excipient, or vehicle with which a compound, presently the cell component, is administered. Pharmaceutically acceptable carriers are preferably sterile liquids, such as water, oils and hydrogels. Water or aqueous solution saline solutions and aqueous dextrose and glycerol solutions are preferably employed as carriers, particularly for injectable solutions. Most preferably hydrogels are employed as carriers, particularly for injectable media. Preferably, such semi-liquid pharmaceutically acceptable carriers include collagen hydrogels and collagen gels and other natural or synthetic polymers.

In a preferred embodiment of the present invention, the pharmaceutically acceptable carrier, in particular in form of a gel or hydrogel, is selected from the group consisting of hyaluronic acid and its derivatives, covalently crosslinked gels, collagens, in particular gelatine, dextran, chitosan, carrageenan, alginates, gum arabic (GA), starches, polyethylene oxide (PEO), poly(ethylene glycol) (PEG), poly(caprolactone) (PCL), poly(lactide) (PLA), polyvinyl pyrollidone (PVP), polyacrylic acid (PAA), polymethacrylate (PMA), carboxymethyl cellulose (CMC) and combinations thereof.

In a preferred embodiment, the pharmaceutically acceptable carrier is hyaluronic acid, preferably in the form of a hydrogel.

In a preferred embodiment of the present invention, the pharmaceutical composition for use in the method of treating diabetes in a human or animal subject matter in need thereof comprises nidogen, in particular nidogen-1, pancreatic islets, and as liquid or semi-liquid pharmaceutically acceptable carrier, a hyaluronic acid hydrogel.

In a preferred embodiment of the present invention, the pharmaceutical composition for use in the method of treating diabetes in a human or animal subject matter in need thereof comprises nidogen, in particular nidogen-1, pancreatic pseudo islets, and as liquid or semi-liquid pharmaceutically acceptable carrier, a hyaluronic acid hydrogel.

In a preferred embodiment of the present invention, the pharmaceutical composition comprises in addition to nidogen, the pharmaceutically acceptable carrier and the at least one cell component, further components such as an immune suppressive component and an oxygenation component.

In a preferred embodiment, the pharmaceutical composition further comprises at least one angiogenic growth factor to stimulate angiogenesis of the transplanted cell component or device. However, it is also preferred that the present pharmaceutical composition does not contain any angiogenic growth factors.

In a particularly preferred embodiment the pharmaceutical composition of the present invention consists of nidogen, in particular nidogen-1, a liquid or semi-liquid pharmaceutically acceptable carrier, in particular hyaluronic acid, and at least one of the cell components as referred to herein, in particular pancreatic islets or pseudo-islets.

The present pharmaceutical composition is preferably transplanted into a human or animal recipient. In a preferred embodiment of the present invention, the pharmaceutical composition is for use in a hypoxic or post-transplant, in particular hypoxic and post-transplant environment.

Preferably, the pharmaceutical composition is transplanted subcutaneously, intramuscular, intraperitoneal, into the portal vein, the liver, the spleen, under the kidney capsule, gastrointestinal wall, testis, thymus, bone marrow, anterior chamber of the eye, cerebral ventricles or the omentum. Particularly preferred transplantation sites are the liver and the omentum, which is a fold of the peritoneum connecting the stomach with other abdominal organs.

In a preferred embodiment of the present invention, the pharmaceutical composition is contained in or on a transplantation device.

In the context of the present invention, a "transplantation device" is a device used or intended to be used for transplanting at least one cell, preferably a group of cells into a recipient, in particular a human or animal recipient. Thus, the present invention relates also to a transplantation device comprising a pharmaceutical composition of the present invention.

In a preferred embodiment of the present invention, the transplantation device is a membrane-encapsulated compartment filled with the pharmaceutical composition of the present invention. Preferably, the membrane is a flexible porous membrane. In a preferred embodiment of the present invention, the transplantation device comprises at least one inlet port. The at least one inlet port serves to fill and exchange the pharmaceutical composition of the present invention. Preferably, the transplantation device comprises at least two, in particular two inlet ports.

Preferably, the flexible porous membrane is semi-permeable to provide essential nutrients to the cells but at the same time prevents the recipient's immune cells from passing into the device and harming the cells contained in the device. This setting allows for glucose and oxygen to stimulate the insulin-producing cells contained in the device while preventing immune system cells from recognizing and destroying the transplanted cells.

In a preferred embodiment of the present invention, the transplantation device is an artificial pancreas.

In a preferred embodiment of the present invention, the transplantation device has a substantially elliptical to rectangular form. This outer form can be further compartmentalized to create a plurality of compartments. This is advantageous in so far as such a design increases the surface area for nutrient and oxygen exchange and also prohibits the formation of large cell aggregates or agglomerations where cells packed in the centre receive less or no nutrients and oxygen and therefore potentially will not survive.

The present transplantation device might be filled with the pharmaceutical composition of the present invention before transplantation. However, it is also possible to transplant the device empty in order for it to be vascularised prior to introducing the pharmaceutical composition. Preferably, the pharmaceutical composition is then introduced into the device with a syringe via the at least one inlet port.

The present invention also relates to methods of treating diabetes, in particular diabetes type I or II, in a human or animal subject in need thereof comprising administering an effective amount of a pharmaceutical composition according to the present invention to the subject in need thereof. Thus, the present invention also relates to the use of a pharmaceutical composition of the present invention to treat diabetes, in particular diabetes type I or II, in a human or animal subject in need thereof.

The present invention also provides a method of treating diabetes, in particular diabetes type I or II, in a human or animal in need thereof comprising transplanting the transplantation device containing the pharmaceutical composition according to the present invention into a subject in need thereof. Thus, the present invention also relates to the use of a transplantation device according to the present invention to treat diabetes, in particular diabetes type I or II, in a human or animal subject in need thereof.

The subclaims disclose further preferred embodiments of the present invention.

The following examples and accompanying figures describe the invention in more detail.

The figures show:
**Figure 1** shows the total insulin released (mU/L) per pseudoislet after 72 hours culture in hypoxic when subjected to a GSIS assay. Released insulin was measured by ELISA. The dosage of 30 µg/ml of NIDI elicits the strongest insulin response of pseudo-islets in a post-transplant, hypoxic environment.
**Figure 2** shows the GSIS Index (described by fold increase in insulin secretion when subjected to 20 mmol/L glucose) after 72 hours culture in hypoxic conditions. The dosage of 30 µg/ml of NIDI elicits the strongest insulin response of pseudo-islets in a post-transplant, hypoxic environment.

### EXAMPLE

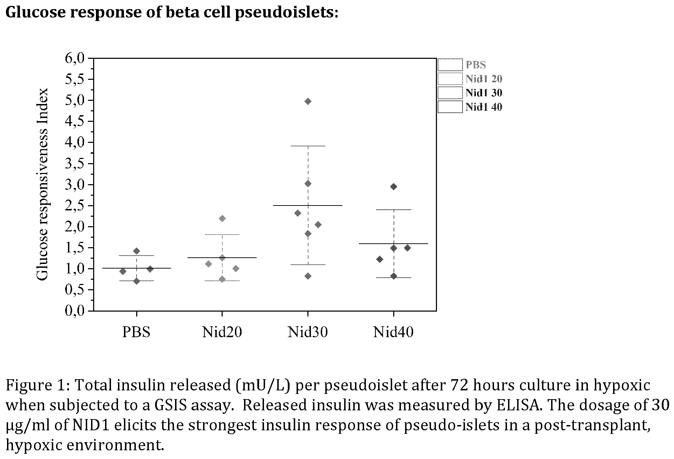

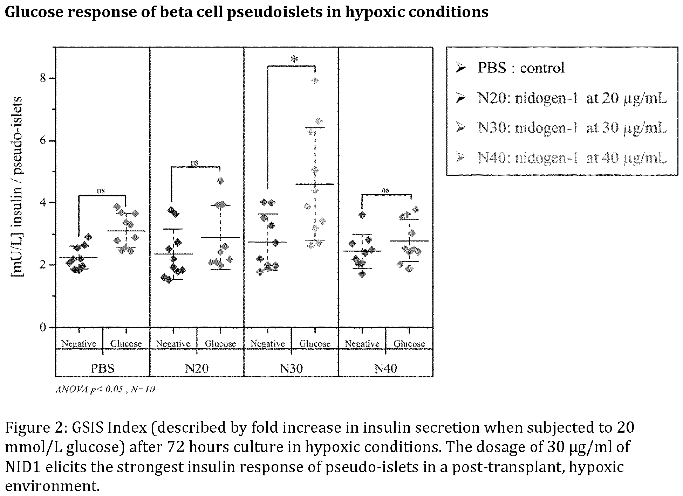

Human pancreatic beta cell pseudo-islets were formed under standard culture conditions at a concentration of 1000 cells/ aggregate for 48h, using 96-well plate with a non-adherent round bottom. After 48h, media was exchanged to fresh media and supplemented using different concentrations of NID1 in the culture: 0 µg/ml, 20 µg/ml, 30 µg/ml and 40 µg/ml. The human pseudo-islets were placed in a hypoxic environment for an additional 72h. Hypoxic conditions are defined as followed: 37°C, 5% CO2 and 1% O₂. After 72h under hypoxia, human pseudo-islets were subjected to a glucose stimulated insulin secretion (GSIS) assay. A concentration of 20 mmol/L glucose was used to stimulate insulin secretion for 1h. The supernatant was collected and insulin measured using the human ultrasensitive ELISA kit (Mercodia). A concentration of 30µg/ml of NIDI in the culture led to an increased insulin release (mU/L) (Figure 1), as well as a statistically relevant strong glucose responsiveness (GSIS index) (Figure 2) in a post-transplant environment when compared to controls and the other conditions.

## Claims

1. A pharmaceutical composition for use in a method of treating diabetes type in a human or animal subject in need thereof comprising nidogen, preferably nidogen-1, a liquid or semiliquid pharmaceutically acceptable carrier and one or more of the cell components pancreatic islets, pancreatic pseudo-islets, pancreatic cells, non-pancreatic insulin producing cells, and pancreatic β-cells, wherein the nidogen is present in a concentration of from 25 µg/ml to 35 µg/ml in the pharmaceutical composition.

2. The pharmaceutical composition according to claim 1, wherein the pharmaceutically acceptable carrier is selected from the group consisting of hyaluronic acid, and its derivatives, covalently crosslinked gels, collagens, preferably gelatine, dextran, chitosan, carrageenan, alginate, gum Arabic (GA), starches, polyethlylene oxide (PEO), poly(ethylene glycol) (PEG), poly(caprolactone) (PCL), poly(lactide) (PLA), polyvinyl pyrollidone (PVP), polyacrylic acid (PAA), polymethacrylate (PMA), carboxymethyl cellulose (CMC) and combinations thereof.

3. The pharmaceutical composition according to claim 1 or 2, wherein the pharmaceutically acceptable carrier is a gel, hydrogel, suspension or solution.

4. The pharmaceutical composition according to anyone of claims 1 to 3, which comprise pancreatic islets, pancreatic pseudo-islets, or both in an amount of 1,000 to 70,000 islet equivalents per kg/bodyweight of the human or animal patient.

5. The pharmaceutical composition according to any one of claims 1 to 3, which comprise 1,000,000 to 90,000,000 pancreatic cells, non-pancreatic insulin producing cells, pancreatic β-cells or combinations thereof per kg/body weight of the human or animal patient.

6. The pharmaceutical composition according to anyone of the preceding claims, further comprising an immune suppressive component and an oxygenation component.

7. The pharmaceutical composition according to anyone of claims 1 to 6, wherein the pharmaceutical composition is contained on or in a transplantation device.

8. A transplantation device comprising a pharmaceutical composition according to anyone of claims 1 to 7.

9. The transplantation device according to claim 8, wherein the transplantation device is a membrane-encapsulated compartment filled with the pharmaceutical composition.

10. The transplantation device according to anyone of claim 9, wherein the membrane is a flexible porous membrane.

11. The transplantation device according to anyone of claims 8 to 10 comprising at least one inlet port.

12. The transplantation device according to anyone of claims 8 to 11, which is an artificial pancreas.

13. A method of treating diabetes in a human or animal subject in need thereof comprising administering an effective amount of a pharmaceutical composition according to anyone of claims 1 to 7 to the subject need thereof.

14. A method of treating diabetes in a human or animal subject in need thereof comprising transplanting a transplantation device according to anyone of claims 8 to 12 into a subject in need thereof.
